# EUROPÄISCHE PATENTANMELDUNG

(11) **EP 4 847 050 A1**
(43) Veröffentlichungstag der Anmeldung: **02.12.2026**
(21) Anmeldenummer: 26179927.4
(22) Anmeldetag: 20.05.2026
(51) Int. Cl.: A61L 2/087, B65B 55/08, A61L 103/00

(54) **VORRICHTUNG UND VERFAHREN ZUM STERILISIEREN VON BEHÄLTNISSEN MITTELS ELEKTRONEN MIT AUS KOHLENSTOFF BESTEHENDEN AUSTRITTSFENSTER**

(30) Priorität: 23.05.2025 DE 102025120285
(71) Anmelder: Krones AG, 93073 Neutraubling (DE)
(72) Erfinder: Kempf, Michael, 93073 Neutraubling (DE)
(74) Vertreter: Hannke Bittner & Partner mbB Regensburg

(57) **Zusammenfassung**

Vorrichtung (1) zum Behandeln und insbesondere Sterilisieren von Behältnissen und insbesondere von Kunststoffvorformlingen (10) und/oder von Verschlüssen (11), insbesondere für Behältnisse (10), mit einer Transporteinrichtung (2), welche dazu geeignet und bestimmt ist, die zu behandelnden Behältnisse (10) und/oder Verschlüsse (11), insbesondere für Behältnisse (10), entlang eines vorgegebenen Transportpfads (T) zu transportieren und mit wenigstens einer ersten Sterilisationsvorrichtung (4), welche dazu geeignet und bestimmt ist, wenigstens eine Oberfläche der Behältnisse (10) und/oder der Verschlüsse (11), insbesondere für Behältnisse (10), zu sterilisieren, wobei diese Sterilisationsvorrichtung (4) eine Ladungsträgererzeugungseinrichtung (42) aufweist, welche Ladungsträger und insbesondere Elektronen erzeugt, sowie eine Unterdruckkammer (44), welche mit einem Unterdruck beaufschlagbar ist sowie eine Beschleunigungseinrichtung (46), welche die von der Ladungsträgererzeugungseinrichtung (42) erzeugten Ladungsträger in Richtung eines Austrittsfensters (48) beschleunigt, wobei dieses Austrittsfenster (48) die Unterdruckkammer gegenüber einer Umgebung (44) abschließt, um den Unterdruck innerhalb der Unterdruckkammer (44) aufrecht zu erhalten, dadurch gekennzeichnet, dass das Austrittsfenster (48) aus einer Kohlenstoff enthaltenden Verbindung besteht.

## Beschreibung

Die vorliegende Erfindung bezieht sich auf eine Vorrichtung und ein Verfahren zum Sterilisieren von Behältnissen und/oder von Verschlüssen, insbesondere für Behältnisse. Derartige Vorrichtungen und Verfahren sind aus dem Stand der Technik seit längerer Zeit bekannt. In jüngerer Zeit ist man dazu bestrebt, nach Möglichkeit auf den Einsatz von chemischen Sterilisationsmitteln zu verzichten oder diesen zumindest zu reduzieren. Daher sind in jüngerer Zeit auch Sterilisationseinrichtungen bekannt geworden, welche die Wandungen der zu sterilisierenden Behältnisse und/oder der Verschlüsse, insbesondere für Behältnisse, mit einer Strahlung und insbesondere einer Ladungsträgerstrahlung und insbesondere mit Elektronen beaufschlagen. Die vorliegende Erfindung wird unter Bezugnahme auf Elektronenstrahler beschrieben, es ist jedoch darauf hinzuweisen, dass eine Anwendung der Erfindung ggf. auch bei anderen Ladungsträgern denkbar ist, wie beispielsweise Protonen oder Alphateilchen.

Ein Problem bei dem Einsatz derartiger Elektronenstrahlern besteht darin, dass die Austrittfenster, über welche die Ladungsträger austreten, sehr empfindlich sind, was sich auch erheblich auf die Laufzeit der Strahlungseinrichtungen auswirkt. Mit anderen Worten ist oftmals der schwächste Teil eines derartigen Strahlungsemitters die das Vakuum zur Elektronenseite abschließende Fensterfolie. Diese muss einerseits sehr dünn sein, so dass die Ladungsträger hindurchtreten können und muss andererseits mechanischen Belastungen, welche beispielsweise durch das Vakuum bei diesen Elektronenstrahlern entstehen, standhalten. Ist eine derartige Folie zerstört, ist die Sterilisationseinrichtung in ihrer Gesamtheit zerstört.

Die Funktion des Austrittsfensters wird derzeit hauptsächlich über Folien aus Titan und Titan in Kombinationen mit anderen Metallen realisiert. Beispiele für derartige Anwendungen ergeben sich beispielsweise aus der EP 0 195 153 A2, der EP 1 374 273 A1 oder der EP 0 480 732 A2.

Die verwendeten Titanfolien sind meist 5-10 µm dick. Die Streuung des Elektronenstrahls bei Transmission und damit der Verlust von Elektronen (Strahlleistung/Flussdichte) für die Anwendung steigt mit der Dicke des Fenstermaterials. Generell gilt, dass umso dünner das Austrittsfenster ist umso weniger Verluste treten auf.

Demzufolge liegt der vorliegenden Erfindung die Aufgabe zugrunde, eine Sterilisationseinrichtung zur Verfügung zu stellen, bei der die Verluste durch das Austrittsfenster geringer sind als im Stand der Technik. Weiterhin soll jedoch das Austrittsfenster eine ausreichende Stabilität aufweisen, um das Vakuum im Inneren der Vakuumkammer, in welcher die Elektronen beschleunigt werden, zu ermöglichen. Daneben soll entstehende Wärme bei der Streuung reduziert werden. Titanfolien brennen bei zu hohem Energieeintrag durch die Elektronen durch. Daher soll auch die Beständigkeit des Austrittsfensters erhöht werden.

Diese Aufgabe wird gemäß den Merkmalen der unabhängigen Patentansprüche gelöst. Vorteilhafte Ausführungsformen und Weiterbildungen sind Gegenstand der Unteransprüche.

Eine erfindungsgemäße Vorrichtung zum Behandeln und insbesondere Sterilisieren von Behältnissen und insbesondere von Kunststoffvorformlingen und/oder von Verschlüssen, insbesondere für Behältnisse, weist eine Transporteinrichtung auf, welche dazu geeignet und bestimmt ist, die zu behandelnden Behältnisse und/oder die zu behandelnden Verschlüsse, insbesondere für Behältnisse, entlang eines vorgegebenen Transportpfads zu transportieren und wenigstens eine erste Sterilisationsvorrichtung, welche dazu geeignet und bestimmt ist, wenigstens eine Oberfläche der Behältnisse und/oder der Verschlüsse, insbesondere für Behältnisse, zu sterilisieren, wobei diese Sterilisationsvorrichtung eine Ladungsträgererzeugungseinrichtung aufweist, welche Ladungsträger und insbesondere Elektronen erzeugt, sowie eine Unterdruckkammer, welche mit einem Unterdruck beaufschlagbar ist, sowie eine Beschleunigungseinrichtung, welche die von der Ladungsträgererzeugungseinrichtung erzeugten Ladungsträger in Richtung eines Austrittsfensters beschleunigt, wobei dieses Austrittsfenster die Unterdruckkammer gegenüber einer Umgebung abschließt, um den Unterdruck innerhalb der Unterdruckkammer aufrecht zu erhalten.

Erfindungsgemäß besteht das Austrittsfenster aus einer Kohlenstoff enthaltenden Verbindung oder weist eine Kohlenstoff enthaltende Verbindung auf oder besteht aus einer Kohlenstoff-Modifikation, bevorzugt Graphen, oder weist eine Kohlenstoff-Modifikation, bevorzugt Graphen, auf. Bevorzugt weist das Austrittsfenster wenigstens eine Graphenlage und besonders bevorzugt eine Vielzahl von Graphenlagen auf, beispielsweise weist das Austrittsfenster bis zu 20 Graphenlagen auf. In einer besonders bevorzugten Ausführungsform weist das Austrittsfenster mindestens 20 Graphenlagen, bevorzugt mindesten 50 Graphenlagen, besonders bevorzugt mindestens 100 Graphenlagen, ganz besonders bevorzugt 150 Graphenlagen und insbesondere bevorzugt mindestens 200 Graphenlagen auf. In einer weiteren besonders bevorzugten Ausführungsform weist das Austrittsfenster maximal 3000 Graphenlagen, bevorzugt maximal 1500 Graphenlagen, besonders bevorzugt maximal 1000 Graphenlagen, insbesondere bevorzugt maximal 700 Graphenlagen und ganz besonders bevorzugt maximal 500 Graphenlagen auf.

Unter einer Kohlenstoff enthaltenden Verbindung werden dabei sowohl reiner Kohlenstoff und insbesondere seine Modifikationen, insbesondere Graphen, verstanden, als auch Zusammensetzungen wie Legierungen, welche zumindest auch Kohlenstoff enthalten, sowie Verbindungen aus Kohlenstoff und wenigstens einem weiteren Element, welches nicht Kohlenstoff ist, mit festem stöchiometrischem Verhältnis. Unter Kohlenstoff-Legierungen werden dabei wiederum sowohl homogene metallische Werkstoffe aus Kohlenstoff mit wenigstens einem Metall verstanden, als auch physikalische Gemische aus Kohlenstoff, bevorzugt aus Kohlenstoff-Modifikationen, und wenigstens einem weiteren Reinstoff, bevorzugt wenigstens einem weiteren Reinstoff in elementarer Form, und insbesondere physikalische Gemische aus Kohlenstoff-Modifikationen.

Die Beschleunigungseinrichtung kann eine in dem Gehäuse angeordnete Anode aufweisen (an welcher Spannung an liegt) und welche ein Loch aufweist, durch welches die Elektronen in Richtung Austrittsfenster beschleunigt werden. Die Beschleunigungsspannung ist bevorzugt die Differenz zwischen der Spannung an der Kathode und der Spannung an der Anode.

Mit anderen Worten besteht zumindest ein Anteil des Austrittsfensters aus Kohlenstoff. Bevorzugt ist der Massenanteil des Kohlenstoffs an dem Austrittsfenster größer als 50%, bevorzugt größer als 60%, bevorzugt größer als 70%, bevorzugt größer als 80%, bevorzugt größer als 85%, bevorzugt größer als 90% und besonders bevorzugt größer als 95% und besonders bevorzugt größer als 98%.

Kohlenstoff weist im Periodensystem der Elemente eine erheblich geringere Ordnungszahl (nämlich 6) auf als Titan (nämlich 22). Damit tritt bei gleicher Dicke des Austrittsfensters eine wesentlich geringere Streuung von Elektronen auf. Daneben ist es auch möglich, das Austrittsfenster dünner auszuführen, was unten genauer ausgeführt wird.

Auf diese Weise ist es auch möglich ggfs. die Beschleunigungsspannung zu reduzieren. Daneben kann auch der Emissionsstrom (Anzahl an freien Elektronen, die zur Beschleunigung zur Verfügung stehen) reduziert werden.

Bevorzugt transportiert die Transporteinrichtung die Behältnisse und/oder die Verschlüsse, insbesondere für Behältnisse, einreihig. Bevorzugt transportiert die Transporteinrichtung die Behältnisse und/oder die Verschlüsse, insbesondere für Behältnisse, wenigstens abschnittsweise entlang eines kreisförmigen Transportpfads.

Bevorzugt transportiert die Transporteinrichtung die Behältnisse und/oder die Verschlüsse, insbesondere für Behältnisse, vereinzelt. Bevorzugt weist die Transporteinrichtung einen bewegbaren und insbesondere drehbaren Träger auf, an dem eine Vielzahl von Halteelementen und insbesondere Greifelementen angeordnet ist, welche jeweils dazu geeignet und bestimmt sind, einzelne Behältnisse und/oder einzelne Verschlüsse, insbesondere für Behältnisse, zu halten. Insbesondere werden die Behältnisse dabei an ihren Mündungen und/oder die Verschlüsse, insbesondere für Behältnisse, an ihrer Wandung gehalten. Bevorzugt transportiert die Transporteinrichtung die Behältnisse und/oder die Verschlüsse, insbesondere für Behältnisse, in wenigstens eine Richtung, wobei die Behältnisse und/oder die Verschlüsse, insbesondere für Behältnisse, zudem rotieren. Bevorzugt rotieren die Behältnisse und/oder die Verschlüsse, insbesondere für Behältnisse, ohne einen Transport in eine Richtung.

Bevorzugt liegt ein Unterdruck innerhalb der Vakuumkammer unterhalb von 10⁻⁶ mbar, bevorzugt unterhalb von 10⁻⁷ mbar. Bevorzugt liegt ein Unterdruck in der Vakuumkammer oberhalb von 10⁻⁹ mbar.

Besonders bevorzugt werden die Elektronen mittels der Beschleunigungseinrichtung mit einer Beschleunigungsspannung beschleunigt welche größer ist als 20kV, bevorzugt größer als 40kV, bevorzugt größer als 50kV, bevorzugt größer als 60kV, bevorzugt größer als 70kV und bevorzugt größer als 80kV und besonders bevorzugt größer als 90kV.

Besonders bevorzugt werden die Elektronen mittels der Beschleunigungseinrichtung mit einer Beschleunigungsspannung beschleunigt, welche kleiner ist als 200kV, bevorzugt kleiner als 150kV, bevorzugt kleiner als 140kV, bevorzugt kleiner als 120kV und bevorzugt kleiner als 110kV und besonders bevorzugt kleiner als 100kV ist.

Eine wesentliche Idee der Erfindung ist die Verwendung einer dünnen Kohlenstoffschicht als Austrittsfenster für Elektronen vom Vakuum zum Druckbereich und das insbesondere im Anwendungsfeld der Sterilisation von Behältnissen und/oder von Verschlüssen, insbesondere für Behältnisse, und insbesondere der PET-Flaschensterilisation und/oder der Sterilisation von PET - Kunststoffvorformlingen. Bevorzugt handelt es sich bei den Verschlüssen um Verschlüsse für Behältnisse und bevorzugt sind diese Verschlüsse dazu geeignet das Behältnis nach einem Füllvorgang mit einem Füllgut, bevorzugt einer Flüssigkeit, insbesondere bevorzugt einem Getränk, zu verschließen. Solche Verschlüsse können auch als Deckel oder Kappen bezeichnet werden und im Rahmen der vorliegenden Erfindung werden diese Begriffe synonym verwendet.

Die Kohlenstoffschicht dichtet hierbei das Ende eines vakuumierten Stabs (unten als stangenartiger Körper bezeichnet) ab, in dem der Elektronenstrahl in oder auf das zu sterilisierende Behältnis und/oder in oder auf den zu sterilisierenden Verschluss, insbesondere für Behältnisse, geführt wird.

Eine Kohlenstofflage, auch Graphen genannt, weist hervorragende Materialeigenschaften auf. Für die Anwendung relevant sind der extrem hohe Young's Modulus (vergleichbar mit Diamanten) als Maß für die Stabilität, daneben die höchste Temperaturleitfähigkeit bzw. eine sehr hohe Wärmeleitfähigkeit zur Abfuhr von über die Elektronen eingebrachte Energie (thermische) und Schichtdicken bis an die physikalischen Grenzen und damit minimaler Elektronenverlust.

Bevorzugt weist die Kohlenstoffschicht wenigstens eine Kohlenstofflage, insbesondere eine Graphenlage auf, bevorzugt eine Vielzahl von Kohlenstofflagen, insbesondere eine Vielzahl von Graphenlagen, besonders bevorzugt bis zu 20 Kohlenstofflagen, insbesondere bis zu 20 Graphenlagen, auf. In einer besonders bevorzugten Ausführungsform weist die Kohlenstoffschicht mindestens 20 Graphenlagen, bevorzugt mindesten 50 Graphenlagen, besonders bevorzugt mindestens 100 Graphenlagen, ganz besonders bevorzugt 150 Graphenlagen und insbesondere bevorzugt mindestens 200 Graphenlagen auf. In einer weiteren besonders bevorzugten Ausführungsform weist die Kohlenstoffschicht maximal 3000 Graphenlagen, bevorzugt maximal 1500 Graphenlagen, besonders bevorzugt maximal 1000 Graphenlagen, insbesondere bevorzugt maximal 700 Graphenlagen und ganz besonders bevorzugt maximal 500 Graphenlagen auf.

Besonders bevorzugt besteht die Kohlenstoffschicht aus wenigstens einer Kohlenstofflage, insbesondere einer Graphenlage, bevorzugt einer Vielzahl von Kohlenstofflagen, insbesondere einer Vielzahl von Graphenlagen, besonders bevorzugt aus bis zu 20 Kohlenstofflagen, insbesondere aus bis zu 20 Graphenlagen. In der vorliegenden Erfindung werden die Begriffe Kohlenstofflage und Graphenlage bzw. Graphen synonym verwendet.In einer besonders bevorzugten Ausführungsform besteht die Kohlenstoffschicht aus mindestens 20 Graphenlagen, bevorzugt mindesten 50 Graphenlagen, besonders bevorzugt mindestens 100 Graphenlagen, ganz besonders bevorzugt 150 Graphenlagen und insbesondere bevorzugt mindestens 200 Graphenlagen. In einer weiteren besonders bevorzugten Ausführungsform besteht die Kohlenstoffschicht aus maximal 3000 Graphenlagen, bevorzugt maximal 1500 Graphenlagen, besonders bevorzugt maximal 1000 Graphenlagen, insbesondere bevorzugt maximal 700 Graphenlagen und ganz besonders bevorzugt maximal 500 Graphenlagen.

Hinzukommend hat Kohlenstoff im Vergleich zu Titan eine kleinere Ordnungszahl (Periodensystem) und damit bei gleicher Dicke einen generell niedrigeren Streuquerschnitt für Elektronen und damit einen intrinsisch geringeren Verlust im Vergleich zu Titan.

Bei einer weiteren bevorzugten Ausführungsform besteht das Austrittsfenster aus Kohlenstoff und weist insbesondere Graphen auf, wobei bevorzugt das Austrittsfenster wenigstens eine und bevorzugt mehrere Kohlenstofflagen aufweist.

Graphen ist die Bezeichnung für eine Modifikation des Kohlenstoffs mit zweidimensionaler Struktur. Jedes Kohlenstoffatom ist im Winkel von 120° über Atombindungen mit drei weiteren verbunden, so dass sich ein bienenwabenförmiges Muster ausbildet. Darüber hinaus ist jedes Kohlenstoffatom auch an einer Doppelbindung beteiligt. Die Elektronen dieser Doppelbindungen, die π-Elektronen, sind jedoch vollständig delokalisiert, das heißt frei über die gesamte Wabenstruktur beweglich. Unter Graphen kann daher eine zweidimensionale Einzellage an sp²-hybridisiertem Kohlenstoffatomen verstanden werden.

Damit handelt es sich bei der Graphenstruktur um eine Verknüpfung sehr vieler Benzolringe zu einer durchgehenden Fläche. Graphen lässt sich damit als Extremfall eines polycyclischen aromatischen Kohlenwasserstoffs beschreiben. Aufgrund der durchgehenden Verknüpfungen in den Flächen sind jedoch dort gar keine Wasserstoffatome gebunden. Nur an den Rändern des Wabengitters sind zum Erreichen der Vierbindigkeit der C-Atome weitere Bindungspartner, beispielsweise Wasserstoffatome, vorhanden. Gemessen an der Größe des Gesamtmoleküls haben sie kaum Einfluss auf die Eigenschaften des Graphens.

Eine besondere Eigenschaft von Graphen ist vor allem dessen Zweidimensionalität. Physikalisch gesehen handelt es sich um eine Schicht aus einzelnen Kohlenstoffatomen bzw. eine einzelne Schicht aus Kohlenstoffatomen, die in einem sechseckigen Muster und/oder einer hexagonalen Kristallstruktur angeordnet sind, das optisch einer Bienenwabe ähnelt. Graphen ist also eine allotrope Variante von Kohlenstoff.

Es hat sich herausgestellt, dass Graphen extrem gut Wärme und Elektrizität leitet. Außerdem zeichnet es sich durch einen geringen aktiven, insbesondere elektrischen, Widerstand aus. In dieser Hinsicht konkurriert es mit Kupfer und Silizium. Es wäre dabei möglich, dass das Austrittsfenster auch als Elektrode für die Beschleunigung der Elektronen dient.

Besonders bevorzugt weist das Austrittsfenster und/oder das kohlenstoffhaltige Material einen Youngschen Modul auf, der größer ist als 0,5 TPa, bevorzugt größer als 0,7TPa und bevorzugt größer als 0,9TPa. So wurde an einer freihängenden Graphenschicht mit einem atomaren Kraftmikroskop (AFM) die nicht lineare Elastizität bestimmt und darüber der Y-oungs Modulus zu E = 1.0 Terapascal abgeleitet. Damit gehört es zu den stärksten bekannten Materialien, mit Diamant.

Der Youngsche Modul ( E ) beschreibt die Zugelastizität oder die Neigung eines Gegenstands sich entlang einer Achse zu verformen, wenn gegenwirkende Kräfte entlang dieser Achse angewendet werden. Er ist definiert als das Verhältnis von Zugspannung zu Zugdehnung . Er wird häufig einfach als Elastizitätsmodul bezeichnet.

Bei einer weiteren bevorzugten Ausführungsform ist die erste Sterilisationseinrichtung dazu geeignet und bestimmt, wenigstens eine Innenoberfläche des Behältnisses und insbesondere eines Kunststoffvorformlings und/oder des Verschlusses, insbesondere für Behältnisse, zu sterilisieren.

Bei einer weiteren bevorzugten Ausführungsform weist das Austrittsfenster wenigstens abschnittsweise eine Dicke auf, welche größer ist als 50nm, bevorzugt größer als 70nm, bevorzugt größer als 100nm, bevorzugt größer als 120nm und besonders bevorzugt größer als 200nm. Bevorzugt weist das Austrittsfenster diese Dicke in einem Bereich auf, in welchem die Elektronen durch das Austrittsfenster austreten. Dabei ist bevorzugt diese Dicke im Wesentlichen konstant.

Bei einer weiteren bevorzugten Ausführungsform weist das Austrittsfenster wenigstens abschnittsweise eine Dicke auf, welche kleiner ist als 3000nm, bevorzugt kleiner als 2000nm, bevorzugt kleiner als 1000nm, bevorzugt kleiner als 800nm und besonders bevorzugt kleiner als 600nm, besonders bevorzugt kleiner als 500nm, bevorzugt kleiner als 400nm, besonders bevorzugt kleiner als 350nm und besonders bevorzugt kleiner als 320nm und besonders bevorzugt kleiner als 310nm und besonders bevorzugt kleiner als 300nm.

Bei einer weiteren besonders bevorzugten Ausführungsform weist das Austrittsfenster wenigstens abschnittsweise eine Dicke auf, welche kleiner ist als 10µm, bevorzugt kleiner als 5µm, bevorzugt kleiner als 1µm, besonders bevorzugt kleiner als 500nm, bevorzugt kleiner als 400nm, besonders bevorzugt kleiner als 350nm und besonders bevorzugt kleiner als 250nm und besonders bevorzugt kleiner als 200nm und besonders bevorzugt kleiner als 160nm.

Damit weist das Austrittsfenster eine erheblich geringere Dicke auf als die im Stand der Technik verwendeten Austrittsfenster. Aufwendige Untersuchungen der Anmelderin haben ergeben, dass es möglich ist, die Dicke des Austrittsfensters um mehr als 90% im Vergleich zu einer 10 µm dicken Titanfolie als Austrittsfenster zu reduzieren.

Bei einer weiteren bevorzugten Ausführungsform weist das Austrittsfenster wenigstens eine Kohlenstofflage, bevorzugt wenigstens zwei übereinander angeordnete Kohlenstofflagen, bevorzugt wenigstens drei übereinander angeordnete Kohlenstofflagen, bevorzugt wenigstens vier übereinander angeordnete Kohlenstofflagen und bevorzugt wenigstens fünf übereinander angeordnete Kohlenstofflagen auf.

Bei einer weiteren bevorzugten Ausführungsform weist das Austrittsfenster höchstens 20 übereinander angeordnete Kohlenstofflagen, bevorzugt höchstens 18 übereinander angeordnete Kohlenstofflagen, bevorzugt höchstens 15 übereinander angeordnete Kohlenstofflagen und bevorzugt höchstens zwölf übereinander angeordnete Kohlenstofflagen und bevorzugt höchstens zehn übereinander angeordnete Kohlenstofflagen und besonders bevorzugt höchstens acht übereinander angeordnete Kohlenstofflagen auf.

Die Anmelderin hat ermittelt, dass diese Anzahlen von Kohlenstofflagen einerseits noch eine ausreichende Stabilität bietet und andererseits einen noch weitgehend ungehinderten Austritt der Elektronen durch das Austrittsfenster ermöglicht.

Ein Festkörper bestehend aus mehreren Graphenlagen kann auch als Graphit bezeichnet werden.

Unter einem Festkörper bestehend aus mehreren Graphenlagen, welcher als Graphit bezeichnet wird, wird hierbei ein Festkörper aus wenigstens mehreren tausend Graphenlagen, bevorzugt mit einem Mosaikwinkel von größer gleich 5° und kleiner gleich 15°, besonders bevorzugt mit einem Mosaikwinkel von größer gleich 3° und kleiner gleich 10°, und einer out-of-plane Kristallinität von wenigstens 60%, bevorzugt wenigstens 75%, besonders bevorzugt wenigstens 90%, insbesondere bevorzugt mit Kristalliten von wenigstens 100 Graphenlagen in out-of-plane Richtung, verstanden.

Insbesondere wird unter einem solchen Festkörper aus wenigstens mehreren hundert Graphenlagen, bevorzugt mit einem Mosaikwinkel von kleiner gleich 5°, besonders bevorzugt mit einem Mosaikwinkel kleiner gleich 3°, insbesondere bevorzugt mit einem Mosaikwinkel kleiner gleich 1°, die Kohlenstoff-Modifikation als Highly-Oriented-Pyrolytic-Graphite, kurz HOPG, verstanden. Bei HOPG sind die einzelnen Graphenlagen in der out-of-plane Richtung im Vergleich zu Graphit nahezu perfekt parallel gestapelt, wodurch out-of-plane Kristallinität von bevorzugt wenigstens 90%, insbesondere bevorzugt mit Kristalliten von wenigstens 100 Graphenlagen, erreicht wird.

Ein Festkörper bestehend aus wenigstens zwei übereinander angeordneten Graphenlagen und höchstens 900 übereinander angeordneten Graphenlagen wird auch als Multilayer-Graphen, kurz MLG, bezeichnet. Im Unterschied zu Graphit und insbesondere HOPG weist Multilayer-Graphen weniger Graphenlagen, bevorzugt einen Mosaikwinkel von größer gleich 5°, insbesondere bevorzugt mit einem Mosaikwinkel von größer gleich 3°, und bevorzugt eine out-of-plane Kristallinität von kleiner gleich 90%, besonders bevorzugt kleiner gleich 75%, ganz besonders bevorzugt kleiner gleich 60%, insbesondere bevorzugt Kristallite von kleiner gleich 100 Graphenlagen in out-of-plane Richtung auf. Die physikalischen und chemischen Eigenschaften, insbesondere die minimale Dicke der gestapelten Lagen, die Härte, die mechanische Festigkeit und die thermische bzw. elektrische Leitfähigkeit, insbesondere die anisotrope thermische bzw. elektrische Leitfähigkeit, von Multilayer-Graphen, Graphit und HOPG sind nicht identisch.

Die Angabe out-of-plane bezieht sich dabei auf eine Richtung senkrecht zu einer in einer Ebene liegenden, einzelnen Graphenlagen, insbesondere auf eine Richtung entlang der c-Achse.

Unter dem Begriff Mosaikwinkel wird die Winkelverteilung der Orientierung einzelner Kristallite, bevorzugt einzelner Kristallitdomänen, insbesondere einzelner Graphenlagen verstanden. Mit anderen Worten ist der Mosaikwinkel ein Maß dafür, wie stark die einzelnen Graphenlagen bei übereinander angeordneter Stapelung in Stapelrichtung bzw. in out-of-plane Richtung gegeneinander verdreht sind. Ein Mosaikwinkel von 0° entspricht einer perfekten Ausrichtung der Lagen in out-of-plane Richtung ohne Verdrehung. Der Mosaikwinkel einer Probe lässt sich aus der Halbwertsbreite, auch FWHM genannt, einer Rocking Curve aufgenommen durch Röntgenbeugungsmessung, auch XRD genannt, an der Probe ableiten.

Unter dem Begriff Kristallinität wird die tatsächliche Kristallinität einer Probe im Verhältnis zur theoretischen 100%-Kristallinität eines Einkristalls verstanden. Die Kristallinität einer Probe lässt sich durch Röntgenbeugungsmessungen, auch XRD genannt, an der Probe ableiten, insbesondere aus einer Linienprofilanalyse.

Die Größe von Kristalliten in out-of-plane Richtung lässt sich unter anderem ebenfalls aus Röntgenbeugungsmessungen, auch XRD genannt, unter Verwendung der Scherrer-Gleichung ableiten.

Bei einer weiteren vorteilhaften Ausführungsform weist das Austrittsfenster eine Stützstruktur auf. Es können in dem Austrittsfenster selbst dickere Bereich vorgesehen sein, welche eine Stützstruktur ausbilden. So wäre es möglich, zunächst ein Austrittsfenster mit einer höheren Dicke herzustellen und anschließend in einigen Bereichen die Dicke zu reduzieren. Bevorzugt sind diese Bereiche mit der geringeren Dicke diejenigen Bereiche, durch welche die Elektronen durch das Austrittsfenster austreten. Die Bereiche mit der höheren Dicke dienen bevorzugt als Stützstrukturen.

Bevorzugt sind die Stützstrukturen nur auf einer Seite des Austrittsfensters vorhanden. Besonders bevorzugt sind die Stützstrukturen auf beiden Seiten des Austrittsfensters vorhanden.

Bevorzugt erhöhen die Stützstrukturen die mechanische Belastbarkeit des Austrittsfensters und besonders bevorzugt erhöhen die Stützstrukturen zudem die Wärmeleitfähigkeit und/oder die Temperaturleitfähigkeit des Austrittsfensters.

Bei einer weiteren bevorzugten Ausführungsform ist das Austrittsfenster an einer Stützstruktur angeordnet ist. So kann beispielsweise ein Stützgitter vorgesehen sein, an welchem das Austrittsfenster angeordnet ist.

Bevorzugt weist die Stützstruktur ein Material ausgewählt aus einer Gruppe, umfassend Kohlenstoff, Kupfer, Kupferlegierungen, Nickel, Gold, Molybdän, Aluminium, Titan, Eisenlegierungen, Silizium, Siliziumnitrid, Siliziumoxid, Aluminium, Aluminiumoxid und Kombinationen davon, auf. Besonders bevorzugt besteht die Stützstruktur aus diesem Material.

Besonders bevorzugt handelt es sich bei der Stützstruktur um ein Stützgitter bzw. eine poröse Membran, welche ausgewählt ist aus der Gruppe, umfassend, Kohlenstoffmembrane, Lacey-Kohlenstofffilme, Kupfernetze, Beryllium-Kupfernetze, Kupfer-Rhodiumnetze, Nickelnetze, Aluminiumnetze, Titannetze, Siliziumnitrid-Lochfilme, Siliziumnitrid-Membrane, ultraflache Siliziumoxidfilme und Kombinationen davon, sowie Kombinationen davon mit Polyvinylformalfolien, insbesondere Kombinationen mit Formvarfolien,

Bevorzugt weist die Stützstruktur eine Dicke von größer gleich 1 µm, bevorzugt größer gleich 10 µm, bevorzugt größer gleich 20 µm auf. Bevorzugt weist die Stützstruktur eine Dicke von kleiner gleich 50 µm, bevorzugt kleiner gleich 40 µm, bevorzugt kleiner gleich 30 µm auf.

In einer alternativen Ausführungsform weist die Stützstruktur eine Dicke von größer gleich 100µm, bevorzugt größer gleich 200µm, bevorzugt größer gleich 300µm, besonders bevorzugt größer gleich 350µm auf. Bevorzugt weist die Stützstruktur eine Dicke von kleiner gleich 2000µm, bevorzugt kleiner gleich 1000µm, bevorzugt kleiner gleich 750µm, besonders bevorzugt kleiner gleich 600 µm auf.

Bevorzugt weist die Stützstruktur eine durchschnittliche Lochgröße von größer gleich 0,05 µm, bevorzugt größer gleich 50 µm, bevorzugt größer gleich 100 µm auf. Bevorzugt weist das Stützgitter eine Lochgröße von kleiner gleich 1000 µm, bevorzugt kleiner gleich 700 µm, bevorzugt kleiner gleich 500 µm auf. Beispielsweise weist ein Stützgitter aus Kupfernetz mit einer definierten Maschenweite von Mesh 300 eine definierte Lochgröße von 63 µm und mit einer definierten Maschenweite von Mesh 50 eine durchschnittliche Lochgröße von 425 µm auf. Beispielsweise weist ein Stützgitter aus einem Lacey-Kohlenstofffilm auf einem Copper Mesh 300 eine durchschnittliche Lochgröße von 100 nm bzw. 0,1 µm auf.

Bevorzugt bezieht sich die durchschnittliche Lochgröße bei gitterartigen Stützstrukturen auf den durchschnittlichen Abstand zweier paralleler Randseiten eines Gitterlochs der gitterartigen Stützstruktur, bevorzugt auf den durchschnittlichen größten Abstand zweier paralleler Randseiten eines Gitterlochs der gitterartigen Stützstruktur. Bevorzugt bezieht sich die durchschnittliche Lochgröße bei porösen bzw. löchrigen Stützstrukturen auf den durchschnittlichen Durchmesser der Poren bzw. Löcher der porösen bzw. löchrigen Stützstruktur.

Bevorzugt ist die Stützstruktur dabei wenigstens mittelbar, bevorzugt unmittelbar, auf wenigstens einer Seite des Austrittsfensters angeordnet.

Bevorzugt bedeckt die Stützstruktur das Austrittsfenster teilflächig, wobei maximal 80%, bevorzugt maximal 60%, besonders bevorzugt maximal 40% und insbesondere bevorzugt maximal 20% der Fläche des Austrittsfenster durch die Stützstruktur bedeckt sind. In einer alternativen Ausführungsform bedeckt die Stützstruktur das Austrittsfenster vollflächig.

Diese Stützstruktur kann dabei beispielsweise als Gitterstruktur und/oder als Fadenkreuz ausgebildet sein.

In einer weiteren alternativen Ausführungsform umfasst die Stützstruktur wenigstens ein Stützelement, bevorzugt eine Vielzahl von nicht unmittelbar miteinander verbundenen Stützelementen. Besonders bevorzugt besteht die Stützstruktur aus wenigstens einem Stützelement, bevorzugt aus einer Vielzahl von nicht unmittelbar miteinander verbundenen Stützelementen. Bevorzugt weist wenigstens ein Stützelement eine Balkenform oder Säulenform auf. Bei einer weiteren bevorzugten Ausführungsform umfasst das Austrittsfenster eine Basisschicht, wobei die Basisschicht eine erste Oberfläche und eine zweite Oberfläche aufweist, wobei die erste Oberfläche und die zweite Oberfläche der Basisschicht einander gegenüber liegen, und wenigstens eine Funktionsschicht und bevorzugt eine Vielzahl von Funktionsschichten. Bevorzugt besteht das Austrittsfenster aus diesen Schichten.

Bevorzugt besteht die eine Basisschicht aus einer Kohlenstoff enthaltenden Verbindung oder weist eine Kohlenstoff enthaltende Verbindung auf oder besteht aus einer Kohlenstoff-Modifikation, bevorzugt Graphen, oder weist eine Kohlenstoff-Modifikation, bevorzugt Graphen, auf. Bevorzugt weist die Basisschicht wenigstens eine Graphenlage und besonders bevorzugt eine Vielzahl von Graphenlagen auf, beispielsweise weist die Basisschicht bis zu 20 Graphenlagen auf. In einer besonders bevorzugten Ausführungsform besteht die eine Basisschicht aus mindestens 20 Graphenlagen, bevorzugt mindesten 50 Graphenlagen, besonders bevorzugt mindestens 100 Graphenlagen, ganz besonders bevorzugt 150 Graphenlagen und insbesondere bevorzugt mindestens 200 Graphenlagen. In einer weiteren besonders bevorzugten Ausführungsform besteht die eine Basisschicht aus maximal 3000 Graphenlagen, bevorzugt maximal 1500 Graphenlagen, besonders bevorzugt maximal 1000 Graphenlagen, insbesondere bevorzugt maximal 700 Graphenlagen und ganz besonders bevorzugt maximal 500 Graphenlagen.

Bevorzugt weist die wenigstens eine Funktionsschicht ein Material ausgewählt aus einer Gruppe auf, umfassend Aluminiumoxide, Siliziumoxide, Siliziumnitride, Bornitride, Molybdänsulfide, Aluminium, Silizium, Kupfer, Silber, Chrom, Nickel, Gold, und Platin. Besonders bevorzugt besteht die Funktionsschicht aus einem Material aus einer Gruppe auf, umfassend Aluminiumoxide, Siliziumoxide, Siliziumnitride, Bornitride, Molybdänsulfide, Aluminium, Silizium, Kupfer, Silber, Chrom, Nickel, Gold, Platin und Kombinationen davon. Bevorzugt besteht die wenigstens eine Funktionsschicht aus einem Material ausgewählt aus dieser Gruppe oder aus einer Kombination von diesen.

Bevorzugt weisen die Basisschicht und die Funktionsschicht unterschiedliche Materialien auf.

Bevorzugt besteht die wenigstens eine Funktionsschicht dabei aus einer homogenen Schicht, insbesondere einer nicht unterbrochenen Schicht, oder einen heterogenen Schicht, insbesondere einer unterbrochenen Schicht. Unter einer homogenen Schicht wird dabei eine durchgängige, geschlossene Schicht verstanden. Unter einer heterogenen Schicht wird dabei eine unterbrochene, nicht geschlossene, bevorzugt löchrige, Schicht verstanden.

Diese Ausführungsformen (Austrittsfenster umfassend wenigstens eine Funktionsschicht) bieten den Vorteil, dass die wenigstens eine Funktionsschicht dabei die Eigenschaften der Basisschicht ergänzt und/oder erweitert, bevorzugt verbessert, etwa in dem die Basisschicht vor chemischen Reaktionen, bevorzugt Oxidation und/oder Reduktion, geschützt oder mechanisch verstärkt, bevorzugt zur Aufrechterhaltung des Vakuums verstärkt, wird.

Bevorzugt ist die wenigstens eine Funktionsschicht an der ersten oder zweiten Oberfläche der Basisschicht, wenigstens mittelbar und bevorzugt unmittelbar, angeordnet. Bevorzugt ist an der ersten und zweiten Oberfläche der Basisschicht, wenigstens mittelbar und bevorzugt unmittelbar, jeweils wenigstens eine Funktionsschicht angeordnet, wobei es sich bei den Funktionsschichten um eine Funktionsschicht der gleichen Art oder um voneinander verschiedene Funktionsschichten handelt. Mit anderen Worten kann die gleiche Art von Funktionsschicht auf beiden Seiten angeordnet werden oder es können zwei Funktionsschichten, welche sich in wenigstens einer Eigenschaft unterscheiden, angeordnet sein. Beispielsweise ist auf einer Seite der Basisschicht eine Schicht zum Schutz der Basisschicht vor Oxidation angeordnet und auf der anderen Seite der Basisschicht eine Schicht zur mechanischen Verstärkung angeordnet. Es wäre auch denkbar, dass eine solche Schicht zum Schutz der Basisschicht vor Oxidation auch auf beiden Seiten der Basisschicht angeordnet ist. Es wäre auch denkbar, dass dieselbe Art von Funktionsschichten auf beiden Seiten der Basisschicht angeordnet ist, aber mit unterschiedlichen Dicken

Das Austrittsfenster kann somit bevorzugt einen heterogenen Lagenaufbau aufweisen, umfassend Basisschicht und wenigstens eine Funktionsschicht. Bevorzugt weist das Austrittsfenster eine Dicke auf, welche größer gleich 50nm, bevorzugt größer gleich 70nm, bevorzugt größer gleich 100nm, bevorzugt größer gleich 120nm und besonders bevorzugt größer gleich 200nm ist. Bevorzugt weist das Austrittsfenster diese Dicke in einem Bereich auf, in welchem die Elektronen durch das Austrittsfenster austreten. Dabei ist bevorzugt diese Dicke im Wesentlichen konstant.

Bei einer weiteren bevorzugten Ausführungsform weist das Austrittsfenster eine Dicke auf, welche kleiner gleich 3000nm, bevorzugt kleiner gleich 2000nm, bevorzugt kleiner gleich 1000nm, bevorzugt kleiner gleich 800nm und besonders bevorzugt kleiner gleich 600nm, besonders bevorzugt kleiner gleich 500nm, bevorzugt kleiner gleich 400nm, besonders bevorzugt kleiner gleich 350nm und besonders bevorzugt kleiner gleich 320nm und besonders bevorzugt kleiner gleich 310nm und besonders bevorzugt kleiner gleich 300nm ist.

Bei einer weiteren besonders bevorzugten Ausführungsform weist das Austrittsfenster wenigstens abschnittsweise eine Dicke auf, welche kleiner ist als 10µm, bevorzugt kleiner als 5µm, bevorzugt kleiner als 1µm, besonders bevorzugt kleiner als 500nm, bevorzugt kleiner als 400nm, besonders bevorzugt kleiner als 350nm und besonders bevorzugt kleiner als 250nm und besonders bevorzugt kleiner als 200nm und besonders bevorzugt kleiner als 160nm.

Bei einer weiteren bevorzugten Ausführungsform weist die Basisschicht höchstens 20 übereinander angeordnete Kohlenstofflagen, bevorzugt höchstens 18 übereinander angeordnete Kohlenstofflagen, bevorzugt höchstens 15 übereinander angeordnete Kohlenstofflagen und bevorzugt höchstens zwölf übereinander angeordnete Kohlenstofflagen und bevorzugt höchstens zehn übereinander angeordnete Kohlenstofflagen und besonders bevorzugt höchstens acht übereinander angeordnete Kohlenstofflagen auf. In einer besonders bevorzugten Ausführungsform weist die Basisschicht mindestens 20 Graphenlagen, bevorzugt mindesten 50 Graphenlagen, besonders bevorzugt mindestens 100 Graphenlagen, ganz besonders bevorzugt 150 Graphenlagen und insbesondere bevorzugt mindestens 200 Graphenlagen auf. In einer weiteren besonders bevorzugten Ausführungsform weist die Basisschicht maximal 3000 Graphenlagen, bevorzugt maximal 1500 Graphenlagen, besonders bevorzugt maximal 1000 Graphenlagen, insbesondere bevorzugt maximal 700 Graphenlagen und ganz besonders bevorzugt maximal 500 Graphenlagen auf.

Bei einer weiteren bevorzugten Ausführungsform weist die erste Sterilisationsvorrichtung einen stangenartigen Körper auf, der wenigstens abschnittsweise über eine Mündung des Behältnisses in das Behältnis und insbesondere einen Kunststoffvorformling und/oder über eine Öffnung des Verschlusses in den Verschluss, insbesondere für Behältnisse, einführbar ist, wobei das Austrittsfenster an diesem stangenartigen Körper angeordnet ist, und bevorzugt an einer Stirnseite dieses stangenartigen Körpers angeordnet ist.

Insbesondere ist der stangenartige Körper geeignet, um in Kunststoffvorformlinge eingeführt zu werden.

Bei einer bevorzugten Ausführungsform ist der Außenquerschnitt des stangenartigen Körpers kleiner als 5cm, bevorzugt kleiner als 4cm, bevorzugt kleiner als 3cm, bevorzugt kleiner als 2,5cm und besonders bevorzugt kleiner als 2,0cm.

Bei einer weiteren bevorzugten Ausführungsform ist der Außenquerschnitt des stangenartigen Körpers größer als 0,5cm, bevorzugt größer als 0,7cm, bevorzugt größer als 1,0cm, bevorzugt größer als 1,2cm.

Bei einer weiteren bevorzugten Ausführungsform weist der stangenartige Körper einen gleichbleibenden bzw. konstanten Querschnitt auf.

Bei einer weiteren bevorzugten Ausführungsform weist der stangenartige Körper eine Länge auf, die größer ist als 4cm, bevorzugt größer als 5cm, bevorzugt größer als 7cm, bevorzugt größer als 8cm.

Bei einer weiteren bevorzugten Ausführungsform weist der stangenartige Körper eine Länge auf, die geringer ist als 20cm, bevorzugt geringer als 18cm, bevorzugt geringer als 16cm, bevorzugt geringer als 14cm.

Bei einer weiteren bevorzugten Ausführungsform weist die erste Sterilisationsvorrichtung, insbesondere zur Sterilisation von Verschlüssen, insbesondere für Behältnisse, keinen solchen stangenartigen Körper auf, und ist nicht dazu geeignet wenigstens abschnittsweise über eine Öffnung des Verschlusses in den Verschluss, insbesondere für Behältnisse, eingeführt zu werden. Mit anderen Worten ist das Austrittsfenster hierbei wenigstens mittelbar, bevorzugt unmittelbar, stirnseitig nach einer Anode (in Richtung des zu sterilisierenden Verschlusses, insbesondere für Behältnisse) einer Beschleunigungseinrichtung angeordnet und nicht am Ende des stangenartigen Körpers, wodurch das Austrittsfenster nicht über eine Öffnung des Verschlusses in den Verschluss, insbesondere für Behältnisse, eingeführt werden kann.

Weiterhin ist bevorzugt eine Antriebseinrichtung vorgesehen, welche dazu geeignet und bestimmt ist, diesen stangenartigen Körper bezüglich des Behältnisses und/oder bezüglich des Verschlusses, insbesondere für Behältnisse, in einer Längsrichtung des Behältnisses und/oder in einer Richtung senkrecht zur Öffnungs- bzw. Mündungsebene des Verschlusses, insbesondere für Behältnisse, und/oder einer Längsrichtung des stangenartigen Körpers zu bewegen, wobei bevorzugt diese Antriebseinrichtung als Linearmotor ausgebildet ist.

Besonders bevorzugt bewegt die Antriebseinrichtung jeweils den stangenartigen Körper, es wäre jedoch auch möglich, dass die Antriebseinrichtung die zu sterilisierenden Behältnisse und/oder die zu sterilisierenden Verschlüsse, insbesondere für Behältnisse, bewegt.

Bei einer weiteren bevorzugten Ausführungsform weist der stangenartige Körper ein Gehäuse auf, welches die Vakuumkammer umgibt. Besonders bevorzugt ist dieses Gehäuse wenigstens abschnittsweise doppelwandig ausgeführt, wobei zwischen diesen Wänden bevorzugt ein fließfähiges Medium und insbesondere ein Kühlmedium führbar ist.

Bevorzugt ist das Gehäuse aus einem Metall und insbesondere aus Aluminium gefertigt.

Bevorzugt ist eine Vielzahl von ersten Sterilisationseinrichtungen vorgesehen, welche bevorzugt jeweils die hier beschriebenen stangenartigen Körper aufweisen. Bevorzugt ist jede dieser ersten Sterilisationseinrichtung jeweils einer Halteeinrichtung zum Halten der Behältnisse zugeordnet, um so den stangenartigen Körper in das jeweils von der Halteeinrichtung gehaltene Behältnis und insbesondere den gehaltenen Kunststoffvorformling einzuführen. Bevorzugt ist jeder dieser ersten Sterilisationseinrichtungen jeweils einer Halteeinrichtung zum Halten der Verschlüsse, insbesondere für Behältnisse, zugeordnet.

Bei einer weiteren vorteilhaften Ausführungsform weist die Vorrichtung eine Kühleinrichtung auf, welche dazu geeignet und bestimmt ist das Austrittsfenster zu kühlen. Wie oben erwähnt ist das hier vorgeschlagene Graphen ein sehr guter Wärmeleiter. Daher könnte beispielsweise das Austrittsfenster mit einem Material in Kontakt stehen, welches ebenfalls ein guter Wärmeleiter ist und dieses Material könnte gekühlt werden. Auch wäre es möglich, dass eine ausreichende Wärmeabfuhr über das Gehäuse erfolgt, an welchem das Austrittsfenster angeordnet ist.

Besonders bevorzugt weist diese Kühleinrichtung eine Beaufschlagungseinrichtung auf, welche dazu geeignet und bestimmt ist das Austrittsfenster zu dessen Kühlung mit einem fließfähigen und insbesondere flüssigen oder gasförmigen Medium zu beaufschlagen. Dabei ist es möglich, dass das Austrittsfenster von außen her mit dem Kühlmedium beaufschlagt wird.

Besonders bevorzugt wird dieses Kühlmittel durch einen Spalt geführt, der zwischen zwei Wandungen des Gehäuses des stangenartigen Körpers gebildet ist.

Bei einer weiteren bevorzugten Ausführungsform weist die Vorrichtung wenigstens eine zweite Sterilisationseinrichtung auf, die geeignet und bestimmt ist, eine weitere Oberfläche der Behältnisse und/oder der Verschlüsse, insbesondere für Behältnisse, und insbesondere eine Außenoberfläche der Behältnisse und/oder der Verschlüsse, insbesondere für Behältnisse, zu sterilisieren. Bevorzugt erfolgt dabei auch die Sterilisation dieser Außenoberfläche mittels Ladungsträgern und insbesondere mittels Elektronen.

Besonders bevorzugt weist diese zweite Sterilisationseinrichtung ein Austrittsfenster aufweist, über welches Ladungsträger aus einer Vakuumkammer austreten können, wobei besonders bevorzugt das Austrittsfenster aus einer Kohlenstoff enthaltenden Verbindung besteht oder eine Kohlenstoff enthaltende Verbindung aufweist oder aus einer Kohlenstoff-Modifikation, bevorzugt Graphen, besteht oder eine Kohlenstoff-Modifikation, bevorzugt Graphen, aufweist. Bevorzugt weist das Austrittsfenster wenigstens eine Graphenlage und besonders bevorzugt eine Vielzahl von Graphenlagen auf, beispielsweise weist das Austrittsfenster bis zu 20 Graphenlagen auf. In einer besonders bevorzugten Ausführungsform weist das Austrittsfenster mindestens 20 Graphenlagen, bevorzugt mindesten 50 Graphenlagen, besonders bevorzugt mindestens 100 Graphenlagen, ganz besonders bevorzugt 150 Graphenlagen und insbesondere bevorzugt mindestens 200 Graphenlagen auf. In einer weiteren besonders bevorzugten Ausführungsform weist das Austrittsfenster maximal 3000 Graphenlagen, bevorzugt maximal 1500 Graphenlagen, besonders bevorzugt maximal 1000 Graphenlagen, insbesondere bevorzugt maximal 700 Graphenlagen und ganz besonders bevorzugt maximal 500 Graphenlagen auf.

Besonders bevorzugt ist diese zweite Sterilisationseinrichtung stationär angeordnet und die zu sterilisierenden Behältnisse und/oder die zu sterilisierenden Verschlüsse, insbesondere für Behältnisse, werden an dieser zweiten Sterilisationseinrichtung vorbeitransportiert.

Die vorliegende Erfindung ist weiterhin auf eine Sterilisationsvorrichtung zum Sterilisieren von Behältnissen und insbesondere von Kunststoffvorformlingen und/oder von Verschlüssen, insbesondere für Behältnisse, gerichtet, welche dazu geeignet und bestimmt ist, wenigstens eine Oberfläche und insbesondere eine Innenoberfläche der Behältnisse und/oder der Verschlüsse, insbesondere für Behältnisse, zu sterilisieren, wobei diese Sterilisationseinrichtung eine Ladungsträgererzeugungseinrichtung aufweist, welche Ladungsträger und insbesondere Elektronen erzeugt, sowie eine Unterdruckkammer, welche mit einem Unterdruck beaufschlagbar ist oder beaufschlagt ist sowie eine Beschleunigungseinrichtung, welche die von der Ladungsträgererzeugungseinrichtung erzeugten Ladungsträger in Richtung eines Austrittsfensters beschleunigt (und/oder welche hierzu geeignet und bestimmt ist), wobei dieses Austrittsfenster die Unterdruckkammer abschließt, um den Unterdruck innerhalb der Unterdruckkammer aufrecht zu erhalten.

Erfindungsgemäß besteht das Austrittsfenster aus einer Kohlenstoff enthaltenden Verbindung oder weist eine Kohlenstoff enthaltende Verbindung auf oder besteht aus einer Kohlenstoff-Modifikation, bevorzugt Graphen, oder weist eine Kohlenstoff-Modifikation, bevorzugt Graphen, auf. Bevorzugt weist das Austrittsfenster wenigstens eine Graphenlage und besonders bevorzugt eine Vielzahl von Graphenlagen auf, beispielsweise weist das Austrittsfenster bis zu 20 Graphenlagen auf. In einer besonders bevorzugten Ausführungsform weist das Austrittsfenster mindestens 20 Graphenlagen, bevorzugt mindesten 50 Graphenlagen, besonders bevorzugt mindestens 100 Graphenlagen, ganz besonders bevorzugt 150 Graphenlagen und insbesondere bevorzugt mindestens 200 Graphenlagen auf. In einer weiteren besonders bevorzugten Ausführungsform weist das Austrittsfenster maximal 3000 Graphenlagen, bevorzugt maximal 1500 Graphenlagen, besonders bevorzugt maximal 1000 Graphenlagen, insbesondere bevorzugt maximal 700 Graphenlagen und ganz besonders bevorzugt maximal 500 Graphenlagen auf.

Bevorzugt besteht das Austrittsfenster aus wenigstens einer und bevorzugt mehreren Lagen Graphen.

Bevorzugt weist die Sterilisationseinrichtung einen stangenartigen Körper auf, der durch eine Mündung in die Behältnisse einführbar ist. Das besagte Austrittsfenster ist bevorzugt in einem Endabschnitt und insbesondere an einer Stirnfläche dieses stangenartigen Körpers angeordnet

Die vorliegende Erfindung ist weiterhin auf ein Verfahren zum Sterilisieren von Behältnissen und insbesondere von Kunststoffvorformlingen und/oder von Verschlüssen, insbesondere für Behältnisse, mittels Ladungsträgern (und insbesondere Elektronen) gerichtet, wobei eine Ladungsträgererzeugungseinrichtung Ladungsträger und insbesondere Elektronen innerhalb einer Unterdruckkammer erzeugt, welche mit einem Unterdruck beaufschlagt ist oder wird, und eine Beschleunigungseinrichtung die von der Ladungsträgererzeugungseinrichtung erzeugten Ladungsträger in Richtung eines Austrittsfensters beschleunigt, wobei dieses Austrittsfenster die Unterdruckkammer abschließt, um den Unterdruck innerhalb der Unterdruckkammer aufrecht zu erhalten.

Erfindungsgemäß besteht das Austrittsfenster aus einer Kohlenstoff enthaltenden Verbindung oder weist eine einen Kohlenstoff enthaltende Verbindung auf oder besteht aus einer Kohlenstoff-Modifikation, bevorzugt Graphen, oder weist eine Kohlenstoff-Modifikation, bevorzugt Graphen, auf. Bevorzugt weist das Austrittsfenster wenigstens eine Graphenlage und besonders bevorzugt eine Vielzahl von Graphenlagen auf, beispielsweise weist das Austrittsfenster bis zu 20 Graphenlagen auf. In einer besonders bevorzugten Ausführungsform weist das Austrittsfenster mindestens 20 Graphenlagen, bevorzugt mindesten 50 Graphenlagen, besonders bevorzugt mindestens 100 Graphenlagen, ganz besonders bevorzugt 150 Graphenlagen und insbesondere bevorzugt mindestens 200 Graphenlagen auf. In einer weiteren besonders bevorzugten Ausführungsform weist das Austrittsfenster maximal 3000 Graphenlagen, bevorzugt maximal 1500 Graphenlagen, besonders bevorzugt maximal 1000 Graphenlagen, insbesondere bevorzugt maximal 700 Graphenlagen und ganz besonders bevorzugt maximal 500 Graphenlagen auf.

Es wird daher auch verfahrensseitig vorgeschlagen, für das Austrittsfenster ein kohlenstoffhaltiges Material und insbesondere Graphen vorzusehen.

Bevorzugt wird wenigstens das Austrittsfenster in die zu sterilisierenden Behältnisse eingeführt, um eine Innenwandung der Behältnisse zu sterilisieren. Bevorzugt ist dabei die Erzeugung und Beschleunigung der Elektronen aktiviert, während ein stangenartiger Körper, an dem das Austrittsfenster angeordnet ist, in die Kunststoffvorformlinge eingeführt wird und/oder während dieser stangenartige Körper wieder aus den Kunststoffvorformlingen herausgezogen wird.

Die vorliegende Erfindung ist weiterhin auf Verwendung einer Kohlenstoffverbindung und insbesondere von Graphen für ein Austrittsfenster einer Sterilisationseinrichtung zum Sterilisieren von Behältnissen und/oder von Verschlüssen, insbesondere für Behältnisse, gerichtet, wobei Elektronen durch dieses Austrittsfenster hindurchtreten können.

Insbesondere wird das Austrittsfenster verwendet um einen Unterdruck innerhalb einer Vakuumkammer, in welcher Elektronen in Richtung des Austrittsfensters beschleunigt werden, aufrecht zu erhalten.

Weitere Vorteile und Ausführungsformen ergeben sich aus den beigefügten Figuren. Darin zeigen:
- Fig. 1: eine Anlage zum Behandeln von Behältnissen;
- Fig. 2: eine schematische Darstellung zur Veranschaulichung der Erfindung;
- Fig. 3: eine erste Ausführungsform einer erfindungsgemäßen Sterilisationseinrichtung;
- Fig. 4: eine schematische Darstellung eines Strahlerfingers einer erfindungsgemäßen Vorrichtung;
- Fig. 5: eine Detaildarstellung eines Strahlerfingers;
- Fig. 6: eine weitere Detaildarstellung eines Strahlerfingers;
- Fig. 7: eine weitere Detaildarstellung eines Strahlerfingers; und
- Fig. 8: eine weitere schematische Darstellung zur Veranschaulichung der Erfindung.

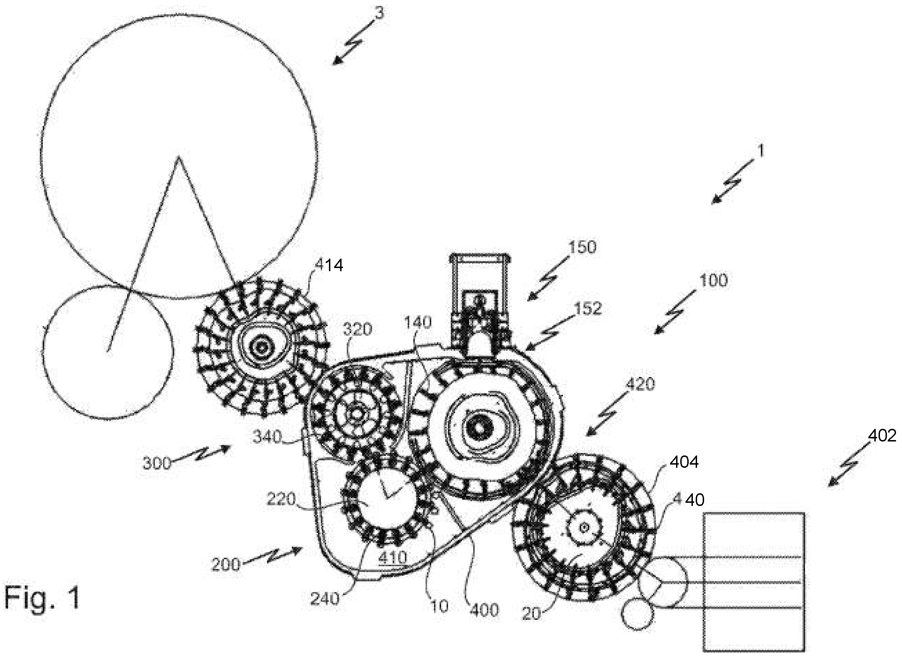

Fig. 1 zeigt eine schematische Aufsicht auf eine Behältnisbehandlungsvorrichtung 1 in einer beispielhaften Ausführungsform. Die gezeigte Behältnisbehandlungsvorrichtung 1, bei der es sich im gezeigten Beispiel um eine Behältnissterilisationsvorrichtung handelt, weist bevorzugt mehrere Transporteinrichtungen 100, 200, 300 zum Transport eines Behältnisses 10, genauer gesagt eines Kunststoffvorformlings entlang eines in dieser Darstellung nicht hervorgehobenen Transportpfads innerhalb eines Gehäuses 400
auf.

Eine dieser Transporteinrichtungen 100, 200, 300 ist eine Behältnisaußenflächenbehandlungseinrichtung 100 oder weist eine solche auf und eine andere eine Behältnisinnenflächenbehandlungseinrichtung 200. Diese weisen jeweils eine Vielzahl von Halteeinrichtungen 140, 240 zum Halten mindestens eines Behältnisses 10 während einer Behältnisbehandlung und/oder des Transports entlang des Transportpfads auf.

Zumindest eine Halteeinrichtung 140 der Behältnisaußenflächenbehandlungseinrichtung 100 kann bevorzugt zumindest zeitweise in den Bereich einer Öffnung 420 der Gehäusewandung 400 gebracht werden. Dadurch wird ermöglicht, dass dieses Halteelement 140 aus einem Raum 412 außerhalb des Gehäuses 400 von einer dort stromaufwärts
bezüglich des Transportpfads angeordneten Transporteinrichtung 404 ein Behältnis aufnehmen kann.

Durch diese Ausgestaltung kann auf eine zusätzliche Transporteinrichtung innerhalb des Gehäuses verzichtet werden, die das Behältnis von außerhalb aufnimmt und an die Behältnisaußenflächenbehandlungseinrichtung 100 weiterleitet.

Die Transporteinrichtung 404 ist in der gezeigten Ausführungsform stromabwärts einer Heizvorrichtung 402 angeordnet, die mindestens eine Heizeinrichtung 5 wie beispielsweise einen Ofen oder eine (beispielsweise Infrarot- oder Mikrowellen-) Strahlungsquelle umfasst.

Die Transporteinrichtung 404 weist zur Aufnahme der Behältnisse 10 bevorzugt eine Vielzahl von Halteelementen 440 auf, die gemeinsam auf einem rotierbaren Träger 20 angeordnet sind.

Bevorzugt ist ein weiterer Teil der dargestellten Behältnisaußenflächenbehandlungseinrichtung 100 eine Behältnisaußenflächenbeaufschlagungseinrichtung 150, die im gezeigten Beispiel eine Strahlungsquelle 150 ist oder aufweist. Denkbar wäre jedoch auch deren Ausgestaltung als Düse oder Düsenanordnung, mittels welcher ein fluides Medium wie eine Sterilisationslösung oder ein sterilisierendes Gas auf die zu sterilisierende Behältnisfläche aufgebracht wird.

In der Behältnisaußenflächenbehandlungseinrichtung 100 werden die Behältnisse durch die Haltelemente 140 in einem Behältnisaußenflächenbeaufschlagungsbereich 152 an der Behältnisaußenflächenbeaufschlagungseinrichtung 150 vorbeigeführt. Während dieses Transports werden sie mit dem sterilisierenden Medium - in diesem Fall sterilisierende Strahlung - beaufschlagt. Vorteilhaft werden die Behältnisse 10 dabei nicht nur entlang des Transportpfads bewegt, sondern wie an anderer Stelle beschrieben ist in mindestens einer weiteren Richtung bewegt. Diese Bewegung kann eine Rotation (um eine Behältnislängsachse und/oder eine Behältnisquerachse) und/oder eine Verschiebung senkrecht zur Zeichenebene (also in Höhenrichtung H) umfassen.

Durch eine solche individuelle Bewegung des zu behandelnden Behältnisses ist es möglich, alle zu sterilisierenden (Außen-) Flächen mit einer einzigen Behältnisaußenflächenbeaufschlagungseinrichtung 150 zu behandeln. Dauerhafte Abschattungen einzelner (Außen-) Oberflächen des Behältnisses 10 durch andere Abschnitte desselben Behältnisses können vermieden werden.

Bei der in dargestellten Ausführungsform wird ein Behältnis 10 nach der Behandlung durch die in Fig 1 gezeigte Behältnisaußenflächenbehandlungseinrichtung 100 unmittelbar an eine Behältnisinnenflächenbehandlungseinrichtung 200 übergeben. Diese umfasst bevorzugt ebenfalls eine Vielzahl von Halteeinrichtungen 240, die an einem rotierenden Träger 220 angeordnet sind.

Ebenso ist bevorzugt eine Behältnisinnenflächenbeaufschlagungseinrichtung vorgesehen. Die Behältnisinnenflächenbeaufschlagungseinrichtung ist in der gezeigten Darstellung nicht deutlich erkennbar, da sie als eine Vielzahl von Strahlfingern ausgeführt ist, die sich jeweils mit einer Position zur Aufnahme eines Behältnisses 10 überdecken.

Jedem Haltelement 240 ist bevorzugt somit ein solcher Strahlfinger zugeordnet. Während der Rotation des Trägers 220 werden die Behältnisse relativ zu demjenigen Strahlfinger (insbesondere in der Längsrichtung der Behältnisse bewegt, der dem von diesem Behältnis 10 besetzten Halteelement 240 zugeordnet ist. Diese Bewegung erfolgt in einem Abschnitt zwischen der Aufnahme deines Behältnisses 10 durch die Behältnisinnenflächenbehandlungseinrichtung 200 und der Abgabe an eine entlang des Transportpfads folgende Transporteinrichtung 300.

Vorzugsweise handelt es sich bei der dieser Transporteinrichtung 300 um einen Transportstern 300 mit einem rotierbaren Träger 320 und einer Vielzahl daran angeordneter Haltelemente 340. Dieser Transportstern 300 nimmt die von der Behältnisinnenflächenbehandlungseinrichtung 200 behandelten Behältnisse 10 auf und übergibt sie an eine außerhalb dieses Gehäuses 400 angeordnete weitere Transporteinrichtung 414.

Vorzugsweise erfolgt auch der weitere Transport unter Bedingungen, die eine Kontamination der durch die Behältnisbehandlungsvorrichtung 1 behandelten Behältnisse 10
verhindert, beispielsweise in einem Reinraum.

Wie in dem gezeigten Beispiel dargestellt ist, ist der vom Gehäuse 400 umschlossene Bereich 410 besonders klein, was unter anderem mit der lediglich einen weiteren Transporteinrichtung 300 zusätzlich zu der Behältnisaußenflächenbehandlungseinrichtung 100 und der Behältnisinnenflächenbehandlungseinrichtung 200 innerhalb des Gehäuses 400 begründet werden kann.

Als der der Behandlung in der Behältnisbehandlungsvorrichtung 1 nachgeschaltete weitere Behandlung der Behältnisse kann, beispielsweise deren Umformung zu anderen Behältnissen 10, wie beispielsweise Flaschen, durch eine lediglich schematisch angedeutete Umformungseinrichtung 3 erfolgen. Ergänzend oder alternativ dazu wäre das Befüllen (und gegebenenfalls Verschließen) der durch die Behältnisbehandlungsvorrichtung 1 behandelten Behältnisse 10 denkbar.

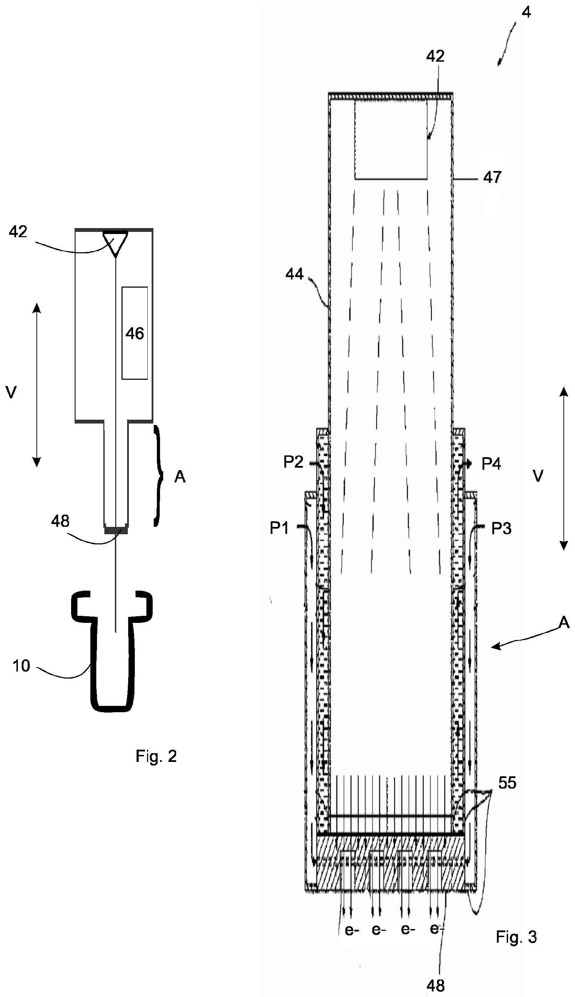

Fig. 2 zeigt eine schematische und stark vereinfachte Darstellung einer Sterilisationseinrichtung. Diese weist eine Elektronenquelle bzw. Ladungsträgererzeugungseinrichtung 42 auf. Diese kann in bekannter Weise einen Glühwendel aufweisen, der durch einen Stromfluss erhitzt wird wobei durch den glühelektrischen Effekt Elektronen aus dem Metall gelöst werden können. Es bildet sich eine Wolke aus freien Elektronen. Das Bezugszeichen 46 kennzeichnet schematisch eine Beschleunigungseinrichtung, welche die Elektronen in Richtung eines Austrittsfensters 48 beschleunigt. Diese Beschleunigungseinrichtung weist bevorzugt eine Anode und eine Kathode auf, welche diese Beschleunigung bewirken.

Eine Beschleunigungsspannung erzeugt ein elektrisches Feld zwischen der Kathode bzw. dem Glühwendel und einer Anode. Dieses beschleunigt die freien Elektronen von der Glühwendel weg in Richtung einer Anode.

Der Bereich A der Sterilisationseinrichtung ist in ein Behältnis 10 wie insbesondere einen Kunststoffvorformling einführbar. Die Sterilisationseinrichtung kann bevorzugt in Richtung des Doppelpfeils V bewegt werden und auf diese Weise kann ein stangenartiger Körper, insbesondere der Bereich A, der Sterilisationseinrichtung in ein Behältnis 10 und insbesondere einen Kunststoffvorformling 10 eingeführt werden.

Fig. 3 zeigt eine weitere Darstellung der Sterilisationseinrichtung 4.

Das Bezugszeichen 48 kennzeichnet das Austrittsfenster, durch welches hindurch die Ladungsträger bzw. Elektronen e- aus der Vakuumkammer austreten können. Das Bezugszeichen 55 kennzeichnet Verbindungsstellen wie beispielsweise Schweißverbindungen mittels denen das Austrittsfenster an der Vakuumkammer bzw. deren Gehäuse befestigt ist.

Bei dieser Ausführungsform ist vorgesehen, dass das Austrittsfenster 48 selbst eine Stützstruktur ausbildet. Man erkennt an der Unterseite des Austrittsfensters 48 vier sich in der vertikalen Richtung erstreckende Schlitze, an deren das Austrittsfenster entsprechend dünner ist und an denen die Elektronen austreten. Andere Bereiche des Austrittsfensters 48 sind erheblich dicker und tragen auf diese Weise zum Stabilisieren des Austrittsfensters bei. Man erkennt weiterhin, dass das Austrittsfenster 48 gekühlt wird. Zu diesem Zwecke kann beispielswese Kühlluft entlang der Seitenwände beispielsweise entlang der Pfeile P1 - P3 nach unten geführt werden und insbesondere an dem Austrittsfenster vorbeigeführt werden

Das Bezugszeichen 48 kennzeichnet ein Austrittsfenster, durch welches die beschleunigten Elektronen aus der Sterilisationseinrichtung austreten können. Die Sterilisationseinrichtung kann bevorzugt in Richtung des Doppelpfeils V bewegt werden und auf diese Weise kann ein stangeartiger Körper der Sterilisationseinrichtung in ein Behältnis 10 und insbesondere einen Kunststoffvorformling 10 eingeführt werden. Die beschleunigten Elektronen, welche aus dem Austrittsfenster austreten, bewirken bevorzugt eine (wenigstens teilweise) Sterilisation der Innenoberflächen des Kunststoffvorformlings 10. Das Bezugszeichen 47 kennzeichnet ein Gehäuse der Sterilisationseinrichtung 4.

Fig. 3 zeigt damit eine detailliertere Darstellung der Sterilisationseinrichtung. Hier ist wieder die Ladungsträgererzeugungseinrichtung 42 erkennbar. Das Bezugszeichen 44 kennzeichnet eine Vakuumkammer, innerhalb derer die Elektronen erzeugt und auch beschleunigt werden.

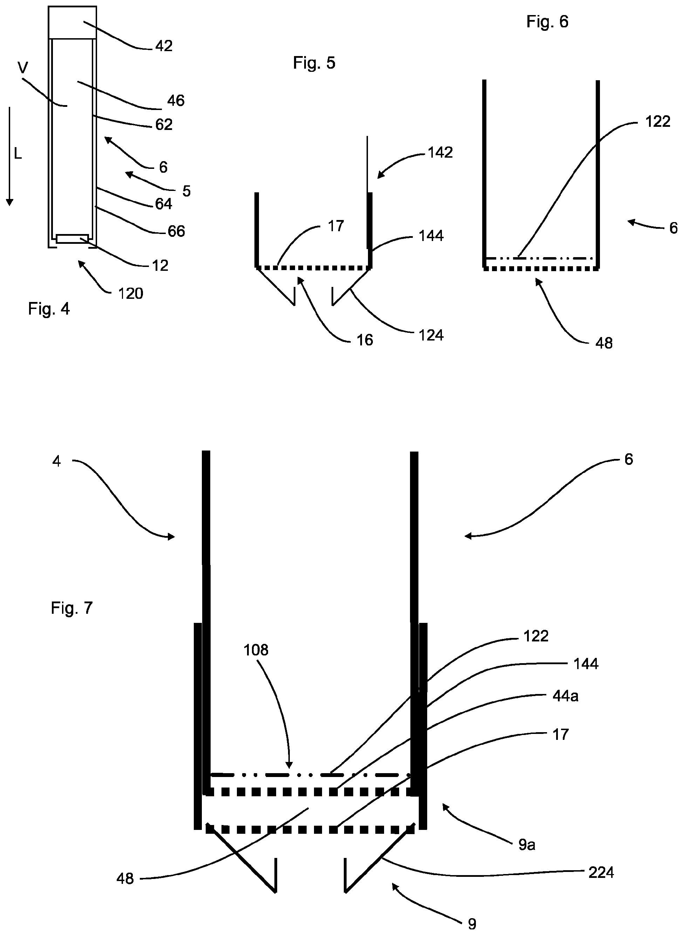

Fig. 4 zeigt eine schematische Darstellung einer Sterilisationseinrichtung in Form eines Strahlfingers 5, der in das Behältnis einführbar ist. Dieser Strahlfinger weist ein Gehäuse 6 auf, welches hier stangenartig ausgebildet ist und sich in einer Längsrichtung L erstreckt.

Diese Längsrichtung L ist dabei üblicherweise auch eine Längsrichtung der Behältnisse, in welche das Gehäuse 6 mit der Austrittseinrichtung 12 einführbar ist. Diese Längsrichtung ist hier auch die Beschleunigungsrichtung, in der die Ladungsträger beschleunigt werden und/oder die Bewegungsrichtung, in der sich die Ladungsträger innerhalb des Strahlfingers 5 bevorzugt bewegen.

Das Bezugszeichen 42 kennzeichnet eine Ladungsträgererzeugungseinrichtung, das Bezugszeichen 44 (nur grob schematisch) eine Beschleunigungseinrichtung, welche die erzeugten Ladungsträger in Richtung der Austrittseinrichtung fördert bzw. beschleunigt.

Das Bezugszeichen 120 bezieht sich grob schematisch auf eine Kühleinrichtung zum Kühlen dieser Austrittseinrichtung bzw. des oben genannten Austrittsfensters. Zu diesem Zweck ist - wie gezeigt - das Gehäuse 6 bevorzugt doppelwandig ausgeführt, wobei die Kühlkanäle für die Kühlungseinrichtungen in dem Zwischenraum 66 zwischen den Wandungen 62 und 64 ausgeführt sind. Das Bezugszeichen V kennzeichnet das (unter einem (Teil-) vakuum stehende) Innenvolumen des Gehäuses.

Fig. 5 zeigt eine schematische Darstellung eines Aufsatzes 142, der hier das zweite Material 17 des Austrittselementes bzw. Austrittsfensters trägt. Hierbei kann es sich um eine Aluminiumfolie handeln. Das Bezugszeichen 144 kennzeichnet einen Grundkörper dieses Aufsatzes, der auf den Grundkörper des Gehäuses 6 (vgl. Fig. 4) aufsetzbar ist.

Das Bezugszeichen 124 kennzeichnet eine Umlenkeinrichtung, welche ein gasförmiges Medium wie insbesondere Kühlluft derart umlenkt, dass sie auf das Material 17 des Austrittsfensters (von außen) trifft. Dabei bewirkt diese Umlenkeinrichtung eine Umlenkung des gasförmigen Mediums um wenigstens 80°, bevorzugt um wenigstens 90°, bevorzugt um wenigstens 120° und bevorzugt um einen Winkel, der zwischen 120° und 170° liegt.

Fig. 6 zeigt eine schematische Darstellung des unteren Abschnitts des Strahlfingers bzw. des Gehäuses 6. Dabei ist hier das Austrittselement 48 d.h. das Austrittsfenster dargestellt, welches ein Kohlenstoffmaterial aufweist. Das Bezugszeichen 122 kennzeichnet eine Stützeinrichtung bzw. Stützstruktur, die das Austrittsfenster 48 nach innen hin abstützt. Auf diese Weise kann das Austrittsfenster 48 besser dem Außendruck standhalten. Es wäre jedoch auch eine Abstützung nach außen hin möglich.

Fig. 7 zeigt eine detailliertere Darstellung der Anordnung aus dem Gehäuse 6 mit dem daran aufgesetzten Aufsatz Innerhalb der Stützeinrichtung 122 kann ein flüssige Kühlmedium geführt werden. Dabei können innerhalb dieser als Stützgitter ausgeführten Stützeinrichtung bzw. des Fensterträgers Kanäle, beispielsweise als Verteilkanäle, zu den einzelnen Stützgitterkanälen ausgebildet sein. Die Führung des Kühlwassers bzw. der Kühlflüssigkeit in diesem Träger bzw. Stützgitter 122 richtet sich bevorzugt nach der Geometrie in den einzelnen Kanälen.

Sie könnten beispielsweise hohldrahtartig gebogen sein, um unterschiedlichste Geometrien einsetzen zu können. Auch wäre es möglich, dass die Stützeinrichtung 122 (die als Gitter ausgeführt sein kann) beispielsweise aus zwei aufeinander aufgeschweißten Teilen bestehen, in die die Kanäle eingefräst sind. Auch wäre es möglich, dass die Kanäle unter Verwendung von Querstreben gebohrt werden.

Bei der in Fig. 7 gezeigten Ausführungsform ist es möglich, dass der Strahlfinger bzw. das Gehäuse 6 über einen so genannten Kühlflansch mit einem Gehäuse der Elektronenerzeugungseinrichtung verbunden ist. Die Medienleitungen werden aus dem Finger bzw. in den Finger durch diesen Kühlflansch geführt und bevorzugt seitlich abgeführt. In diesem Fall kann auf eine Durchführung der Leitungen durch das Hochvakuumgeneratorgehäuse verzichtet werden.

Zwischen dem Grundkörper 144 des Aufsatzes 142 und dem Gehäuse 6 bzw. dem Strahlfinger kann ein Kanal ausgebildet sein, durch den hindurch das Vakuum erzeugt wird. Auch kann (in der Figur nicht dargestellt) eine Zuführeinrichtung wie etwa ein Kanal vorgesehen sein, der das gasförmige Medium an die Umlenkeinrichtung 124 und damit von außen her an das Material 17 des Austrittsfensters führt. Ein derartiger Kanal kann auch in einer Wandung des Aufsatzes 42 ausgeführt sein.

Das Bezugszeichen 9 bezieht sich auf die erste Beaufschlagungseinrichtung in ihrer Gesamtheit, welche das erste Austrittsfenster (hier mit einem flüssigen) Medium beaufschlagt. Wesentlicher Bestandteil dieser ersten Beaufschlagungseinrichtung 9 ist die oben erwähnte Stützeinrichtung 122, die von dem flüssigen Medium durchflossen werden kann.

Diese erste Beaufschlagungseinrichtung 9 kühlt damit bevorzugt einen ersten Bereich der Austrittseinrichtung genauer des ersten Austrittselements 48. Eine zweite Beaufschlagungseinrichtung kühlt einen zweiten Bereich der Austrittseinrichtung 48. Es wird darauf hingewiesen, dass auch nur eine der genannten Kühleinrichtungen vorgesehen sein kann. Dies ist insbesondere bei dem im Rahmen der Erfindung vorgeschlagenen Austrittsfenster bevorzugt, da sich dieses aufgrund seines Materials und seiner Materialdicke deutlich weniger erwärmt als die im Stand der Technik verwendeten Titanfenster.

Das Bezugszeichen 9a kennzeichnet eine zweite optionale oder alternative Beaufschlagungseinrichtung, welche hier beispielsweise die Umlenkeinrichtung 24 aufweist. Diese zweite Beauschlagungseinrichtung 9a beaufschlagt vorzugsweise Austrittsfenster unmittelbar mit dem gasförmigen Medium zu dessen Kühlung.

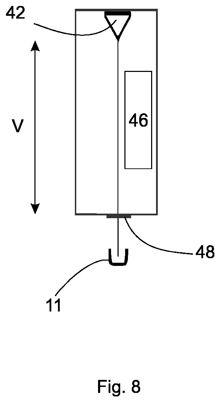

Fig. 8 zeigt eine schematische und stark vereinfachte Darstellung einer Sterilisationseinrichtung. Diese weist eine Elektronenquelle bzw. Ladungsträgererzeugungseinrichtung 42 auf. Diese kann in bekannter Weise einen Glühwendel aufweisen, der durch einen Stromfluss erhitzt wird wobei durch den glühelektrischen Effekt Elektronen aus dem Metall gelöst werden können. Es bildet sich eine Wolke aus freien Elektronen. Das Bezugszeichen 46 kennzeichnet schematisch eine Beschleunigungseinrichtung, welche die Elektronen in Richtung eines Austrittsfensters 48 beschleunigt. Diese Beschleunigungseinrichtung weist bevorzugt eine Anode und eine Kathode auf, welche diese Beschleunigung bewirken.

Eine Beschleunigungsspannung erzeugt ein elektrisches Feld zwischen der Kathode bzw. dem Glühwendel und einer Anode. Dieses beschleunigt die freien Elektronen von der Glühwendel weg in Richtung der Anode.

Im Vergleich zur Fig. 2 fehlt hierbei der Bereich A der Sterilisationseinrichtung, welcher in ein Behältnis wie insbesondere einen Kunststoffvorformling einführbar ist. Die Sterilisationseinrichtung eignet sich deshalb insbesondere zur Sterilisation von Verschlüssen 11, insbesondere für Behältnisse.

Es wird darauf hingewiesen, dass sämtliche Merkmale, welche unter Bezugnahme auf das Verfahren beschrieben wurden, in entsprechender Weise auch für die Vorrichtung offenbart sind, was insbesondere bedeutet, dass die entsprechende Vorrichtung Einrichtungen aufweist, welche zur Durchführung der jeweiligen Verfahren geeignet und bestimmt sind. Weiterhin sind auch Merkmale, welche unter Bezugnahme auf die Vorrichtung beschrieben wurden entsprechend für das oder die Verfahren anwendbar. Dies bedeutet, dass die Verfahren unter Verwendung der entsprechenden Vorrichtungsmerkmale ausgeführt werden.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

## Patentansprüche

1. Vorrichtung (1) zum Behandeln und insbesondere Sterilisieren von Behältnissen und insbesondere von Kunststoffvorformlingen (10) und/oder von Verschlüssen (11), insbesondere für Behältnisse (10), mit einer Transporteinrichtung (2), welche dazu geeignet und bestimmt ist, die zu behandelnden Behältnisse und/oder die zu behandelnden Verschlüsse (11), insbesondere für Behältnisse (10), entlang eines vorgegebenen Transportpfads (T) zu transportieren und mit wenigstens einer ersten Sterilisationsvorrichtung (4), welche dazu geeignet und bestimmt ist, wenigstens eine Oberfläche der Behältnisse (10) und/oder der Verschlüsse (11), insbesondere für Behältnisse (10), zu sterilisieren, wobei diese Sterilisationsvorrichtung (4) eine Ladungsträgererzeugungseinrichtung (42) aufweist, welche Ladungsträger und insbesondere Elektronen erzeugt, sowie eine Unterdruckkammer (44), welche mit einem Unterdruck beaufschlagbar ist sowie eine Beschleunigungseinrichtung (46), welche die von der Ladungsträgererzeugungseinrichtung (42) erzeugten Ladungsträger in Richtung eines Austrittsfensters (48) beschleunigt, wobei dieses Austrittsfenster (48) die Unterdruckkammer gegenüber einer Umgebung (44) abschließt, um den Unterdruck innerhalb der Unterdruckkammer (44) aufrecht zu erhalten,
**dadurch gekennzeichnet, dass**
das Austrittsfenster aus einer Kohlenstoff enthaltenden Verbindung besteht.

2. Vorrichtung (1) nach Anspruch 1,
**dadurch gekennzeichnet, dass**
das Austrittsfenster aus Kohlenstoff besteht und insbesondere Graphen aufweist, wobei bevorzugt das Austrittsfenster wenigstens eine und bevorzugt mehrere Kohlenstofflagen aufweist.

3. Vorrichtung (1) nach wenigstens einem der vorangegangenen Ansprüche,
**dadurch gekennzeichnet, dass**
die erste Sterilisationseinrichtung (4) dazu geeignet und bestimmt ist, wenigstens eine Innenoberfläche des Behältnisses (10) und insbesondere eines Kunststoffvorformlings und/oder des Verschlusses (11), insbesondere für Behältnisse (10), zu sterilisieren.

4. Vorrichtung (1) nach wenigstens einem der vorangegangenen Ansprüche,
**dadurch gekennzeichnet, dass**
das Austrittsfenster wenigstens abschnittsweise eine Dicke aufweist, welche größer ist als 50nm, bevorzugt größer als 70nm, bevorzugt größer als 100nm, bevorzugt größer als 120nm und besonders bevorzugt größer als 130nm und/oder das das Austrittsfenster wenigstens abschnittsweise eine Dicke aufweist, welche kleiner ist als 3000nm, bevorzugt kleiner als 2000nm, bevorzugt kleiner als 1000nm, bevorzugt kleiner als 800nm und besonders bevorzugt kleiner als 600nm, besonders bevorzugt kleiner als 500nm, bevorzugt kleiner als 400nm, besonders bevorzugt kleiner als 300nm und besonders bevorzugt kleiner als 250nm und besonders bevorzugt kleiner als 200nm und besonders bevorzugt kleiner als 180nm.

5. Vorrichtung (1) nach wenigstens einem der vorangegangenen Ansprüche,
**dadurch gekennzeichnet, dass**
das Austrittsfenster eine Stützstruktur aufweist und/oder dass das Austrittsfenster an einer Stützstruktur angeordnet ist.

6. Vorrichtung (1) nach wenigstens einem der vorangegangenen Ansprüche,
**dadurch gekennzeichnet, dass**
die erste Sterilisationsvorrichtung (4) einen stangenartigen Körper (52) aufweist, der wenigstens abschnittsweise über eine Mündung des Behältnisses in das Behältnis und/oder über eine Öffnung des Verschlusses (11) in den Verschluss (11), insbesondere für Behältnisse (10), einführbar ist, wobei das Austrittsfenster an diesem stangenartigen Körper angeordnet ist und bevorzugt an einer Stirnseite dieses stangenartigen Körpers (52) angeordnet ist.

7. Vorrichtung (1) nach wenigstens einem der vorangegangenen Ansprüche,
**dadurch gekennzeichnet, dass**
die Vorrichtung (1) eine Kühleinrichtung aufweist, welche dazu geeignet und bestimmt ist das Austrittsfenster zu kühlen, wobei besonders bevorzugt diese Kühleinrichtung eine Beaufschlagungseinrichtung aufweist, welche dazu geeignet und bestimmt ist das Austrittsfenster zu dessen Kühlung mit einem fließfähigen und insbesondere flüssigen oder gasförmigen Medium zu beaufschlagen.

8. Vorrichtung (1) nach wenigstens einem der vorangegangenen Ansprüche,
**dadurch gekennzeichnet, dass**
die Vorrichtung wenigstens eine zweite Sterilisationseinrichtung aufweist, die geeignet und bestimmt ist, eine weitere Außenoberfläche der Behältnisse und/oder der Verschlüsse (11), insbesondere für Behältnisse (10), zu sterilisieren, wobei bevorzugt diese zweite Sterilisationseinrichtung ein Austrittsfenster aufweist, über welches Ladungsträger aus einer Vakuumkammer austreten können, wobei besonders bevorzugt das Austrittsfenster aus einer Kohlenstoff enthaltenden Verbindung besteht.

9. Vorrichtung (1) nach wenigstens einem der vorangegangenen Ansprüche,
**dadurch gekennzeichnet, dass**
das Austrittsfenster aus Graphen mit höchstens 900 Graphenlagen, bevorzugt höchstens 600 Graphenlagen und besonders bevorzugt höchstens 400 Graphenlagen, oder aus Highly Oriented Pyrolytic Graphite mit höchstens 3000 Graphenlagen, bevorzugt höchstens 1500 Graphenlagen, besonders bevorzugt höchstens 1000 Graphenlagen, insbesondere bevorzugt höchstens 700 Graphenlagen und ganz besonders bevorzugt höchstens 500 Graphenlagen, besteht.

10. Vorrichtung (1) nach wenigstens einem der vorangegangenen Ansprüche,
**dadurch gekennzeichnet, dass**
das Austrittsfenster eine Basisschicht, welche eine erste Oberfläche und eine zweite Oberfläche aufweist, wobei die erste Oberfläche und die zweite Oberfläche der Basisschicht einander gegenüber liegen, und wenigstens eine Funktionsschicht umfasst, wobei die Basisschicht und die wenigstens eine Funktionsschicht unterschiedliche Materialien aufweisen.

11. Vorrichtung (1) nach wenigstens einem der vorangegangenen Ansprüche,
**dadurch gekennzeichnet, dass**
das Austrittsfenster eine Stützstruktur aufweist.

12. Sterilisationsvorrichtung zum Sterilisieren von Behältnissen und insbesondere von Kunststoffvorformlingen (10) und/oder Verschlüssen (11), insbesondere für Behältnisse (10), welche dazu geeignet und bestimmt ist, wenigstens eine Oberfläche der Behältnisse (10) und/oder der Verschlüsse (11), insbesondere für Behältnisse (10), zu sterilisieren, wobei diese Sterilisationseinrichtung (4) eine Ladungsträgererzeugungseinrichtung (42) aufweist, welche Ladungsträger und insbesondere Elektronen erzeugt, sowie eine Unterdruckkammer (44), welche mit einem Unterdruck beaufschlagbar ist sowie eine Beschleunigungseinrichtung (46), welche die von der Ladungsträgererzeugungseinrichtung (42) erzeugten Ladungsträger in Richtung eines Austrittsfensters (48) beschleunigt, wobei dieses Austrittsfenster die Unterdruckkammer (44) abschließt, um den Unterdruck innerhalb der Unterdruckkammer (44) aufrecht zu erhalten,
**dadurch gekennzeichnet, dass**
das Austrittsfenster (48) aus einer Kohlenstoff enthaltenden Verbindung besteht.

13. Verfahren zum Sterilisieren von Behältnissen und insbesondere von Kunststoffvorformlingen und/oder Verschlüssen (11), insbesondere für Behältnisse (10), mittels Ladungsträgern wobei eine Ladungsträgererzeugungseinrichtung (42) Ladungsträger und insbesondere Elektronen innerhalb einer Unterdruckkammer erzeugt, welche mit einem Unterdruck beaufschlagt ist oder wird, und eine Beschleunigungseinrichtung (46) die von der Ladungsträgererzeugungseinrichtung (42) erzeugten Ladungsträger in Richtung eines Austrittsfensters (48) beschleunigt, wobei dieses Austrittsfenster die Unterdruckkammer (44) abschließt, um den Unterdruck innerhalb der Unterdruckkammer (44) aufrecht zu erhalten,
**dadurch gekennzeichnet, dass**
das Austrittsfenster (48) aus einer Kohlenstoff enthaltenden Verbindung besteht oder eine einen Kohlenstoff enthaltende Verbindung aufweist.

14. Verfahren nach dem vorangegangenen Anspruch,
**dadurch gekennzeichnet, dass**
das Austrittsfenster in die zu sterilisierenden Behältnisse und/oder die zu sterilisierenden Verschlüsse (11), insbesondere für Behältnisse (10), eingeführt wird, um eine Innenwandung der Behältnisse zu sterilisieren.

15. Verwendung einer Kohlenstoffverbindung und insbesondere von Graphen für ein Austrittsfenster einer Sterilisationseinrichtung zum Sterilisieren von Behältnissen, wobei Elektronen durch dieses Austrittsfenster hindurchtreten können.
